# EUROPEAN PATENT APPLICATION

(11) **EP 1 923 043 A1**
(43) Date of publication of application: **21.05.2008**
(21) Application number: 06023806.0
(22) Date of filing: 16.11.2006
(51) Int. Cl.: A61K 8/49, A61K 8/41, A61K 8/73, A61Q 5/00, A61Q 5/12

(54) **Anti-dandruff conditioner composition**

(71) Applicant: KPSS-Kao Professional Salon Services GmbH, 64297 Darmstadt (DE)
(72) Inventor: Hoffmann, Martin, 64673 Zwingenberg (DE)

(57) **Abstract**

The present invention is related to a anti-dandruff conditioner composition for hair and scalp comprising at least one conditioning compound preferably a cationic conditioning compound and ketocanozol.

## Description

The present invention is related to a anti-dandruff conditioner composition for hair and scalp.

Anti-dandruff cleansing compositions have been known for many years. The compositions are based on surfactants comprising conditioning agents for hair and anti-dandruff agent for removing scalp effectively in a short period of time. As antidandruff agents are anti-mycotic agents such pyrithione salts, piroctone olamine, benzalkonium chloride, ketoconazol, etc.

Leave-in scalp lotions have also been known which are as a rule directly applied to the clap and has very limited, if any, contact to hair body.

Conditioning composition used after cleansing hair is not known. In a hair care practice conditioners as after cleansing compositions are especially for female consumers with relatively longer hair very important for improving combability, elasticity, shine, etc. of hair.

Inventor of the present invention has surprisingly found out if an anti dandruff cleansing composition is used in combination with a conditioner composition comprising an anti-dandruff agent the effectiveness is increased. In other words, scalp becomes more effectively and quickly dandruff free.

Accordingly, the subject of the present invention is a composition for hair and scalp comprising at least one cationic conditioning compound and at least one antidandruff agent ketoconazol.

Another subject of the present invention is the use of a composition comprising at least one hair conditioning compound, preferably a cationic conditioning compound and at least one antidandruff agent preferably ketoconazol as antidandruff composition for scalp and hair.

Although the term scalp is clear and has its obvious meaning, within the meaning of the present invention it is meant skin of head. Although antidandruff conditioning compositions of the present invention is directed particularly to scalp, the use on haired skin is certainly understood as well.

Still further object of the present invention is process for treating hair and scalp wherein a composition comprising at least one conditioning agent, preferably a cationic conditioning agent, and ketoconazol is applied onto wet hair and scalp and after processing for 30 sec to 30 min at a temperature in the range of 20 to 40°C, rinsed off from hair.

Conditioner composition of the present invention can also be offered as a kit together with a cleansing composition with or without antidandruff agent. Accordingly another subject matter of the present invention is a kit for hair and scalp including a cleansing composition based on at least one surfactant with or without antidandruff agent and a conditioning composition comprising at least one conditioning agent and ketoconazol.

Compositions of the present invention comprise at least one antidandruff agent. Most preferred is ketoconazol which may also be mixed with other antidandruff agents so far compatibility between ketoconazol and the other antidandruff agent and also between additional antidandruff agent and conditioning agent present allows. Further antidandruff agents may be piroctone olamine, pyrithione and its metal salts and benzalkonium chloride.

Compositions of the present invention comprise at least one conditioning agent. Conditioning agents are oil and oily substances, non-ionic substances, cationic amphiphilic substances and cationic polymers.

Compositions of the present invention comprise preferably at least one cationic surfactant as a conditioning agent.

As a rule any cationic surfactant is suitable for the compositions of the present invention. With the term cationic surfactant it is meant that the surfactant carries a cationic charge when used in the compositions. In other words, compounds having no cationic charge but when added into the compositions protonate and therewith become cationic are also included within the definition of cationic surfactant. Examples to such may be stearyldimethylamine and PEG-2-Cocamide and the compounds alike. They are as a compound not carrying a cationic charge but when used in a composition having an acidic pH protonates and becomes cationic.

Preferably at least one cationic surfactant is selected from the compounds with the general formula 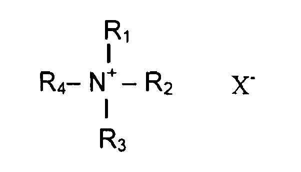 where R₁ is a saturated or unsaturated, branched or non-branched alkyl chain with 8-22 C atoms or

R₅CO NH (CH₂)ₙ

where R₅ is saturated or unsaturated, branched or non-branched alkyl chain with 7-21 C atoms and n has typical value of 0 - 4 or

R₆CO O (CH₂)ₙ

where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or

R₅CO NH (CH₂)ₙ

where R₅ is saturated or unsaturated, branched or non-branched alkyl chain with 7-21 C atoms and n has typical value of 0 - 4 or

R₆ CO O (CH₂)ₙ

where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Suitable cationic surfactants as conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, dimethyl dihydrogenated tallow ammonium chloride, stear trimonium chloride, stearamidopropyldimethylamoonium chloride, dipalmitoyl dimonium chloride, distearyl dimethyl ammonium chloride, stearamidopropyl trimonuim chloride.

Further examples to the cationic surfactants are so called esterquats available on the market, for example, under the trade names "Schercoquat^{®}", "Dehyquart^{®} F30" and "Tetranyl^{®}". Still further examples are so called amidoquatsAgain available on the market, for example, under the trade name "INCROQUAT^{a} HO" or "OCS".

Further cationic conditionic agents are cationic polymers. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has especially been found suitable those cationic polymers known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28, Polyquaternium 30, Polyquaternium 36, Polyquaternium 37, Polyquaternium 46, Polyquaternium 67.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

In this context, reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

Compositions of the present invention comprise oils and/or non-ionic substances as hair conditioning agents. Oils as conditioners according to the present invention are selected from silicone oils either volatile or non-volatile, natural and synthetic oils. Among silicone oils those can be added to the compositions include dimethicone, dimethiconol, polydimethylsiloxane, DC fluid ranges from Dow Corning, cyclosiloxanes such as DC 245, arylated silicones such as phenyltrimethicone available from Dow Corning under trade name DC 556. Synthetic oils include mineral oil such as paraffin oil and petrolatum.

Natural oils suitable are such as olive oil, almond oil, avocado oil, weizenkeim oil, ricinus oil, coconut oil, palm oil, sesame oil, peanut oil, whale oil, sunflower oil, peach kernel oil, wheat germ oil, macadamia nut oil, night primrose oil, jojoba oil, castor oil, or soya oil, lanolin and the derivatives thereof.

Lipophilic oily compounds such as fatty acid esters are as well suitable for the composition of the present invention. Those are such as isopropyl myristate, palmitate, stearate and isostearate, oleyl oleate, isocetyl stearate, hexyl laurate, dibutyl adipate, dioctyl adipate, myristyl myristate, oleyl erucate, cetyl palmitate, etc.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₇ CO (OCH₂CH₂)ₙ OH

R₇ CO (OCH₂CH₂)ₙ O OC R₈

where R₇ and R₈ are independent from each other saturated, unsaturated or branched or non-branched alkyl chain with 7 to 21 C atoms and n is typically 2-100. Typical concentration range for any of the additional conditioners mentioned above other than cationic conditioning compounds can be in the range of 0.01 to 15% by weight, preferably 0.05 - 10% by weight, more preferably 0.1 - 5% by weight calculated to the total composition.

Conditioning agents are comprised in compositions of the present invention at a concentration of 0.01 to 10%, preferably 0.05 to 7.5%, more preferably 0.1 to 5% by weight calculated to total composition. Within the meaning of the present invention it is also possible to use various conditioning agents as a mixture.

Compositions of the present invention can be in the form of a thin liquid, emulsion, thickened liquid and gel.

With the term thickened liquid, it is meant that the compositions comprise additionally a thickening agent.

With the term gel it is meant that the compositions comprise additionally a gelling agent and the gelling agent is a polymer forming a shear thinning gel.

The thickening agents include any polymer either natural or synthetic thickening aqueous composition. Examples are cellulose and its derivatives such as hydroxyethylcellulose, guar and its derivatives such as hydroxypropyl guar. In the selection of the thickening gels compatibility with cationic surfactant should be carefully examined.

The gelling agents include polymers either synthetic or natural forming shear thinning compositions. Examples to the natural polymers are xanthan gum and its derivatives. Synthetic shear thinning polymers may be those of acrylate polymers wherein compatibility with cationic surfactant should carefully be examined prior to use.

Concentration of the thickening and/or gelling agents should be in the range of 0.05 to 5%, preferably 0.1 to 2.5% by weight calculated to total content. It should also be noted that gelling and thickening polymers can be used together.

Emulsion is the preferred form according to the present invention. Emulsions comprise at least one emulsifier. It should be noted that quaternary ammonium compounds with single alkyl chain mentioned above are preferred as emulsifiers as well.

In addition to the cationic surfactants with single alkyl chain, additional emulsifier can be incorporated into the compositions. These additional emulsifiers are surface active substances such as non-ionic, amphoteric or zwitterionic and anionic compounds. Because of the compatibility issues of the anionic surfactants and cationic surfactants mentioned above, anionic surfactants are less suitable and therefore their compatibility should carefully be examined. Otherwise, the compositions of the present invention preferably should substantially be free of anionic surfactants. Preferred emulsifiers are cationic, non-ionic, amphoteric or zwitterionic surfactants. The most preferred additional emulsifiers are non-ionic surfactants.

Suitable non-ionic surfactants are alkyl polyglucosides of the general formula

R₉ - O - (R₁₀O)ₙ O - Zₓ

wherein R₉ is an alkyl group with 8 to 18 carbon atoms, R₁₀ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

Among the non-ionic surfactants mentioned above fatty alcohol ethoxylates are the most preferred ones. Above mentioned non-ionic surfactants can also be used as mixture of one category such as several ethoxylated fatty alcohols or several categories such as mixture of alkyl polyglucoside and ethoxylated fatty alcohol.

As further surfactant component as emulsifier, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate.

Additional emulsifier content of the compositions according to present invention is in the range of 0.05 to 10%, preferably 0.1 to 7.5% and more preferably 0.25 to 5% by weight calculated to total composition.

Emulsions according to the present invention preferably comprise at least one fatty alcohol with linear of branched alkyl chain. Suitable ones are fatty alcohols having 12 to 22 C atoms in its alkyl chain. Examples are myristyl alcohol, cetyl alcohol, stearyl alcohol, behenyl alcohol and their mixtures. Preferred are cetyl, stearyl and behenyl alcohol and their mixtures i.e. cetearyl alcohol. Fatty alcohols may be included into the compositions of the present invention at a concentration of 0.1 to 20%, preferably 0.5 to 15% and more preferably 1 to 10% by weight calculated to total composition.

The compositions according to the present invention can also comprise further agents, such as protein hydrolyzates and polypeptides, e.g. keratin hydrolyzates, collagen hydrolyzates of the type "Nutrilan" or elastin hydrolyzates, as well as, in particular vegetable, optionally cationized protein hydrolyzates, for example "Gluadin".

Additional natural plant extracts can as well form part of the compositions of the present invention. Those are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, green tea, blue lotus flower, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapone" products and "Herbasol^{R} ". Extracts and the preparation thereof are also described in "Hagers Handbuch der pharmazeutischen Praxis", 4^{th} Ed.

The compositions may contain organic solvents such as ethanol. propanol, isopropanol, benzyl alcohol, benzyloxyethanol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the composition should not exceed 5% by weight. It should be noted that penetration enhancers are useful for both cleansing and after shampoo conditioning preparations. It is obvious that the concentration in the cleansing compositions is usually lower than in the conditioning preparations.

Compositions of the present invention can comprise UV filters either for stabilization of the product colour or for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzylidenebornane-2-one and the salts thereof and/or 3-(4'-methyl benzylidene)-DL-campher, polysilicone-15. The preferred amount of the UV-absorber ranges from about 0.01 % to 2.5%, more preferably from 0.05 % to 1 % by weight, calculated to the total composition.

The compositions of the present invention can comprise hair-restructuring agents. The hair restructuring agents preferred are especially the ones disclosed in the German patent DE 197 51 550 C2. Namely they are ceramide type of compounds, fatty acids and phytosterol or their mixtures.

Preferred ceramide compound is cetyl-PG-hydroxyethylpalmitamide.

Preferred fatty acids are those with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and especially 0.01 to 1% by weight calculated to the total weight of the composition. Phytosterol concentration of the conditioners is less than 1% and preferably in the range of 0.01 to 0.5% by weight calculated to the total weight of the composition. It should be noted without limiting the use of those ingredients the effect of those hair restructuring ingredients is especially elevated when used in combination with penetration enhancers.

The pH of the compositions according to the invention is in the range of 2 to 7, preferably 3 to 6, more preferably 3 to 5. For adjusting the pH of the said compositions, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that composition reaches the desired pH value as given above. Typically concentration for acids can be 0.01 - 3% by weight, preferably 0.05 - 2% by weight, more preferably 0.05 - 1.5% by weight calculated to the total composition. The pH of the composition can also be adjusted to the required pH by using alkaline solution such as sodium hydroxide, ammonium hydroxide, potassium hydroxide or their salts with those acids mentioned above in the case that at the selected acid concentration pH of the composition is lower than that of the aimed value.

Compositions of the present invention can comprise additional ingredients having scalp benefits such as soothing, vitalizing, calming, and/or antifat effects. Suitable antifat agents are Zinc salt of pyrrolidoncarboxylic acid (PCA) and 5-α-Avocuta which is butyl esters of fatty acids of avocado oil.

Furthermore, conditioning compositions of the present invention can comprise all substances customarily found in such preparations.

Examples of such substances are complexing agents, dyestuffs, preservatives, fragrances, moisturizers, etc.

Following examples are to illustrate the invention but not to limit.

### Example 1

| | % by weight |
|---|---|
| Ceterayl alcohol | 8.0 |
| Behentrimonium chloride | 2.0 |
| Dimethicone | 0.2 |
| Ketoconázol | 1.0 |
| Lactic acid/Sodium hydroxide | q.s to pH 4.0 |
| Fragrance preservative | q.s. |
| Water | q.s to 100 |

The above composition was prepared by emulsifying fatty alcohol and cationic surfactant in part of water at approximately 70°C and after cooling down to approximately 60°C ketoconazol was mixed and after further cooling down to around 40°C the remaining ingredients were added and pH adjusted to 4.0.

Above compositions was tested with 10 volunteers, mainly female and declared to have dandruff problem for approximately 4 weeks after each shampooing. All were asked not to use antidandruff shampoo during the use of the above conditioner. In face to face interviews after the end of 4 week period 5 volunteers said that they have no dandruff and 3 said the dandruff level is reduced but still had some dandruff and 2 said no change in dandruff level.

From the above it is clear that only use of the above conditioner removes or effectively reduces the dandruff level.

### Example 2

| | % by weight |
|---|---|
| Ceterayl alcohol | 5.0 |
| Polyquaternium-10 | 1.0 |
| Ceteareth 20 | 2.0 |
| Cetrimonium chloride | 1.0 |
| Amodimethicone | 0.5 |
| Ketoconazol | 1.0 |
| Lecithin | 0.1 |
| Panthenol | 0.5 |
| Benzophenone-3 | 0.2 |
| Lactic acid/Sodium hydroxide | q.s to pH 3.5 |
| Fragrance preservative | q.s. |
| Water | q.s to 100 |

The above composition was prepared in the way similar to as described for example 1 except that Polyqauternium 10 was dissolved in a part of water at around 70°C prior to addition.

Similar results were observed after use of the above conditioner with a shampoo composition without antidandruff agent.

In addition, the above conditioner was tested with 10 volunteers having dandruff in a period of 3 weeks. All users were asked to use an antidandruff shampoo comprising 0.4% piroctone olamine. In face to face interviews after the end of 3 week period 8 volunteers declared that they do not have dandruff problem anymore, 2 reported that the dandruff amount was reduced.

### Example 3

| | % by weight |
|---|---|
| Ceterayl alcohol | 6.0 |
| Ceteareth 20 | 2.0 |
| Cetrimonium chloride | 1.5 |
| Guar hydroxypropyltrimonium chloride | 0.5 |
| Ketoconazol | 1.2 |
| Polysilicone-9 | 0.5 |
| Propylene glycol | 0.5 |
| Octylmethoxycinnamate | 0.3 |
| Lactic acid/Sodium hydroxide | q.s to pH 3.8 |
| Fragrance preservative | q.s. |
| Water | q.s to 100 |

The above composition was prepared in a similar way as in example 1 except that Guar hydroxypropyltrimonium chloride was dissolved in a part of water at around 70°C prior to addition.

## Claims

1. Composition for hair and scalp **characterised in that** it comprises at least one cationic conditioning compound and at least one antidandruff agent ketoconazol.

2. Composition according to claim 1 **characterised in that** it comprises additional antidandruff agent.

3. Composition according to claims 1 and 2 **characterised in that** it comprises as a cationic conditioning agent at least one cationic surfactant according to formula 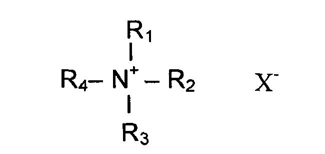 where R₁ is a saturated or unsaturated, branched or non-branched alkyl chain with 8-22 C atoms or
R₅CO NH (CH₂)ₙ
where R₅ is saturated or unsaturated, branched or non-branched alkyl chain with 7-21 C atoms and n has typical value of 0 - 4 or
R₆CO O (CH₂)ₙ
where R₆ is saturated or unsaturated, branched or non-branched alkyl chain with 7-21 C atoms and n has typical value of 0 - 4, and
R₂ is a hydrogen, saturated or unsaturated, branched or non-branched alkyl chain with 1-22 C atoms or
R₅CO NH (CH₂)ₙ
where R₅ is saturated or unsaturated, branched or non-branched alkyl chain with 7-21 C atoms and n has typical value of 0 - 4 or
R₆ CO O (CH₂)ₙ
where R₆ is saturated or unsaturated, branched or non-branched alkyl chain with 7-21 C atoms and n has typical value of 0 - 4,
and R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

4. Composition according to any of the preceding claims **characterised in that** it comprises at least one cationic polymer as conditioning agent.

5. Composition according to any of the preceding claims **characterised in that** it comprises at least one additional conditioning agent selected from oils, oily substances and non-ionic substances.

6. Composition according to any of the preceding claims **characterised in that** it comprises at least one emulsifier selected from cationic, non-ionic and amphoteric surfactants.

7. Composition according to any of the preceding claims **characterised in that** it comprises at least one fatty alcohol.

8. Composition according to any of the preceding claims **characterised in that** it comprises thickening and/or gelling agent.

9. Composition according to any of the preceding claims **characterised in that** it comprises at least one organic solvent.

10. Composition according to any of the preceding claims **characterised in that** it comprises at least one UV filter.

11. Composition according to any of the preceding claims **characterised in that** it comprises at least one scalp benefit agent such as soothing, vitalizing, calming and/or anti-fat agents.

12. Composition according to any of the preceding claims **characterised in that** it has a pH between 2 to 7.

13. Use of a composition according to claims 1 to 12 for treating hair and scalp.

14. Process for treating hair and scalp wherein a composition according to claims 1 to 12 is applied onto wet hair and scalp and after processing for 30 sec to 30 min at a temperature in the range of 20 to 40°C, rinsed off from hair.

15. Kit for hair and scalp including a cleansing composition based on at least one surfactant with or without antidandruff agent and a conditioning composition according to claims 1 to 12.
